# EUROPÄISCHE PATENTANMELDUNG

(11) **EP 0 857 475 A2**
(43) Veröffentlichungstag der Anmeldung: **12.08.1998**
(21) Anmeldenummer: 98101802.1
(22) Anmeldetag: 03.02.1998
(51) Int. Cl.: A61K 6/00

(54) **Zahnprothesenhaftmittel**

(30) Priorität: 05.02.1997 DE 19704293
(71) Anmelder: BASF AKTIENGESELLSCHAFT, 67056 Ludwigshafen (DE)
(72) Erfinder: Schehlmann, Volker, Dr., 67354 Römerberg (DE); Dieing, Reinhold, Dr., 67105 Schifferstadt (DE); Sanner, Axel, Dr., 67227 Frankenthal (DE)

(57) **Zusammenfassung**

Zahnprothesenhaftmittel enthaltend
a) als haftaktiven Wirkstoff teilweise oder vollständig neutralisierte Copolymerisate aus
   5 bis 95 Gew.-%, bezogen auf das Copolymerisat, eines oder mehrerer Acrylester der Formel I

      CH₂=CR'―COOR I (Monomer A),

      in der R' Wasserstoff oder Methyl und R einen Alkylrest mit 1 bis 30 C-Atomen bedeutet und
   95 bis 5 Gew.-% Methacrylsäure und/oder Acrylsäure (Monomer B) und
b) einen in Zahnprothesenhaftmitteln üblichen Träger.

## Beschreibung

Die Erfindung betrifft Zahnprothesenhaftmittel, die als haftaktiven Wirkstoff zumindest teilweise neutralisierte Copolymerisate aus Acryl- oder Methacrylsäureester und Acryl- oder Methacrylsäure zusammen mit an sich bekannten Trägern enthalten.

Haftmittel für Zahnprothesen werden dazu verwendet, das künstliche Gebiß an die Schleimhaut der Gaumenweichteile und an die Zahnfleischfurchen mit dichtem und haftendem Sitz anzupassen oder zumindest die Anpassung zu verbessern. Dabei wird das Haftmittel auf das befeuchtete Gebiß aufgetragen, welches dann in den Mund eingesetzt wird. Der Mundspeichel befeuchtet die Oberfläche der Haftmittelschicht und bringt so das Haftmittel zum Quellen, wodurch auch die Klebekraft entwickelt wird.

Maßgebende Größen sind Haftkraft und Haftdauer. Dabei sind die Mechanismen, die für die Haltewirkung verantwortlich sind, sehr komplex. Die Viskosität der Trägergrundsubstanz und als Folge davon des fertigen Produktes spielen eine wichtige Rolle. Als Trägergrundsubstanz werden u.a. Mineralöl (Paraffinöl), Vaseline (Petrolatum) und Wachse verwendet, die bei Bedarf mit Polyethylenglykol oder Glycerin verdünnt werden und insgesamt ca. 20 bis 60 % des fertigen Produktes ausmachen. Die Viskosität ist primär durch die Gesamtrezeptur vorgegeben. d.h. durch die Gesamtheit der Wirk- und Trägerstoffe im Haftmittel. Es gibt eine große Anzahl von Zusammensetzungen, die jeweils auf unterschiedliche Anwendungen abgestimmt sind.

Sehr gute Haftkraft zeigen die aus zahlreichen Patentschriften, z.B. EP-A 0 122 481 und EP-A 0 265 916 bekannten Zahnprothesenhaftmittel, die als haftaktive Massen Copolymerisate aus Vinylmethylethern und Maleinsäure, die teilneutralisiert oder vollständig neutralisiert sind, enthalten. Als Salze kommen dabei Alkali-, Erdalkali- oder Zinksalze in Betracht.

Die Herstellung dieser Copolymerisate erfolgt allerdings in der Regel in einem organischen Lösungsmittel, das unter großem technischen Aufwand wieder abgetrennt werden muß. Dennoch gelingt die Abtrennung häufig nicht quantitativ, so daß unter Umständen Lösemittelreste in den Endprodukten verbleiben Dies ist unerwünscht.

Aus US-A 4,542,168 ist ferner bekannt, als haftaktive Wirkstoffe teilneutralisierte und vernetzte Polyacrylsäure zusammen mit zumindest einem hydrophilen Polymer zu verwenden. Diese Zahnprothesenhaftmittel haben den Nachteil, daß ihre Haftkraft noch nicht vollständig befriedigt und daß sie ebenfalls in organischen Lösungsmitteln hergestellt werden, die noch im Produkt nachweisbar sind.

Es bestand nun die Aufgabe, Zahnprothesenhaftmittel vorzuschlagen, die als Wirkstoffe Copolymerisate enthalten, die die Haftkraft der Zahnprothesenhaftmittel weiter erhöhen und die bevorzugt lösungsmittelfrei hergestellt werden können.

Die Aufgabe wurde mit Zahnprothesenhaftmitteln gelöst, die
a) als die Haftung aktivierende Masse teilweise oder vollständig neutralisierte Copolymerisate aus
   5 bis 95 Gew.-%, bezogen auf das Copolymerisat, eines oder mehrerer Acrylester der Formel I

      CH₂=CR'―COOR I (Monomer A),

      in der R' Wasserstoff oder Methyl und R einen Alkylrest mit 1 bis 30, vorzugsweise 1 bis 4 C-Atomen bedeutet und
   95 bis 5 Gew.-% Methacrylsäure und/oder Acrylsäure (Monomer B) und
b) einen in Zahnprothesenhaftmitteln üblichen Träger, enthalten.

Bevorzugte, die Haftung aktivierende Massen (a) sind solche aus 20 bis 80 Gew.-% Ethylacrylat oder Methylmethacrylat und 80 bis 20 Gew.-% Methacrylsäure oder Acrylsäure. Im einzelnen seien z.B. Copolymerisate aus ungefähr 50 Gew.-% Ethylenacrylat und ungefähr 50 Gew.-% Acrylsäure z.B. Eudragit® L (Röhm) oder Kollicoat® MAE 30 (BASF) bzw. Copolymerisate aus ungefähr 67 Gew.-% Methylmethacrylat und 33 Gew.-% Methacrylsäure z.B. Eudragit® S (RÖHM) genannt.

Die Copolymere (a) können noch untergeordnete Mengen z.B. bis zu 30 Gew.-%, bezogen auf das Copolymerisat, an weiteren Monomeren C, wie Acrylamide, Methacrylamide, N-Vinyllactame, Hydroxialkylacrylsäureester, Crotonsäure oder Maleinsäure einpolymerisiert enthalten.

Die Copolymere (a) müssen zumindest teilneutralisiert sein. In der Regel sind 30 bis 95 Mol.-%, vorzugsweise 30 bis 80 Mol.-% und besonders bevorzugt 35 bis 75 Mol.-% der freien Carboxylgruppen des Copolymerisats zu den Alkali-, Erdalkali- oder Zinksalzen neutralisiert. Die Salze können einheitlich oder Mischsalze sein. Bevorzugt sind Natrium, Calcium, Magnesium, Strontium oder Zinksalze.

Die Copolymeren haben in der Regel ein Molekulargewicht von 30.000 bis 5 Mio , vorzugsweise 100.000 bis 3 Mio Dalton, so daß sie als zumindest teilneutralisierte Salze wasserlöslich oder zumindest wasserquellbar sind. Das Eigenschaftsprofil d. h. insbesondere die Viskosität kann durch das Herstellverfahren sowie die optimale Salzauswahl gesteuert werden, wobei ein höherer Anteil an zweiwertigen Metallen in der Regel zu einer erhöhten Lösungsmittelviskosität führt.

Die Herstellung der Copolymeren erfolgt in an sich bekannter Weise durch Lösungspolymerisation oder bevorzugt durch Suspensions- oder Emulsionspolymerisation in Wasser. Besonders bevorzugt ist die Emulsionspolymerisation.

Bei der im folgenden näher beschriebenen Emulsionspolymerisation werden ionische und/oder nichtionische Emulgatoren und/oder Schutzkolloide bzw. Stabilisatoren als grenzflächenaktive Verbindungen verwendet.

Eine ausführliche Beschreibung geeigneter Schutzkolloide finden sich in Houben-Weyl, Methoden der organischen Chemie, Band XIV/1, Makromolekulare Stoffe, Georg Thieme Verlag, Stuttgart, 1961, S. 411 bis 420. Als Emulgatoren kommen sowohl anionische, kationische als auch nichtionische Emulgatoren in Betracht. Vorzugsweise werden als begleitende grenzflächenaktive Substanzen ausschließlich Emulgatoren eingesetzt, deren Molekulargewichte im Unterschied zu den Schutzkolloiden üblicherweise unter 2000 g/mol liegen. Selbstverständlich müssen im Falle der Verwendung von Gemischen grenzflächenaktiver Substanzen die Einzelkomponenten miteinander verträglich sein, was im Zweifelsfall anhand weniger Vorversuche überprüft werden kann. Vorzugsweise werden anionsche und nichtionische Emulgatoren als grenzflächenaktive Substanzen verwendet. Gebräuchliche begleitende Emulgatoren sind z.B. ethoxylierte Fettalkohole (EO-Grad: 3 bis 50, Alkylrest; C₈- bis C₃₆), ethoxylierte Mono-, Di- und Tri-Alkylphenole (EO-Grad: 3 bis 50, Alkylrest: C₄- bis C₉), ethoxylierte Sorbitanester (EO Grad: 5 bis 30; gesättigte und ungesättigte C₁₂-C₃₀ Fettsäuren), ethoxylierte Rizinusöle (EO Grad: 5 bis 80, hydriert und unhydriert), Alkalimetallsalze von Dialkylestern der Sulfobernsteinsäure sowie Alkali- und Ammoniumsalze von Alkylsulfaten (Alkylrest; C₈- bis C₁₂), von ethoxylierten Alkanolen (EO-Grad: 4 bis 30, Alkylrest: C₁₂- bis C₁₈), von ethoxylierten Alkylphenolen (EO-Grad: 3 bis 50, Alkylrest: C₄- bis C₉), von Alkylsulfonsäuren (Alkylrest: C₁₂- bis C₁₈) und von Alkylarylsulfonsäuren (Alkylrest: C₉- bis C₁₈).

Weitere geeignete Emulgatoren sind Verbindungen der allgemeinen Formel II 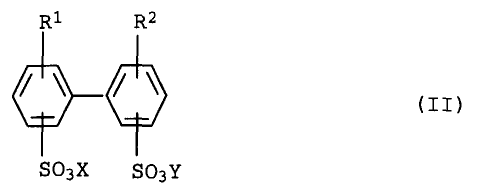 worin R¹ und R² Wasserstoff oder C₄- bis C₁₄-Alkyl bedeuten und nicht gleichzeitig Wasserstoff sind, und X und Y Alkalimetallionen und/oder Ammoniumionen sein können. Vorzugsweise bedeuten R¹, R² lineare oder verzweigte Alkylreste mit 6 bis 18 C-Atomen oder Wasserstoff und insbesondere mit 6, 12 und 16 C-Atomen, wobei R¹, R² nicht beide gleichzeitig Wasserstoff sind. X und Y sind bevorzugt Natrium, Kalium oder Ammoniumionen, wobei Natrium besonders bevorzugt ist. Besonders vorteilhaft sind Verbindungen II in denen X und Y Natrium, R¹ ein verzweigter Alkylrest mit 12 C-Atomen und R² Wasserstoff oder R¹ ist. Häufig werden technische Gemische verwendet, die einen Anteil von 50 bis 90 Gew.-% des monoalkylierten Produktes aufweisen, beispielsweise Dowfax® 2A1 (Warenzeichen der Dow Chemical Company).

Geeignete Emulgatoren finden sich auch in Houben-Weyl, Methoden der organischen Chemie, Band 14/1, Makromolekulare Stoffe, Georg Thieme Verlag, Stuttgart, 1961, Seiten 192 bis 208.

Handelsnamen von Emulgatoren sind z.B. Dowfax® 2A1, Emulan® NP 50, Dextrol® OC 50, Emulgator 825, Emulgator 825 S, Emulan® NP OG, Texapon® NSO, Nekanil® 904 S, Lumiten® I-RA, Lumiten® E 3065, Diponil® FES 77, Lutensol® AT 18, Steinapol® VSL, Emulphor® NPS 25, Cremophor® RH 40, Tween® 80.

Die grenzflächenaktive Substanz wird üblicherweise in Mengen von 0,1 bis 10 Gew.-%, bezogen auf die zu polymerisierenden Monomeren verwendet.

Wasserlösliche Initiatoren für die Emulsionspolymerisation sind z.B. Ammonium- und Alkalimetallsalze der Peroxidischwefelsäure, z.B. Natriumperoxodisulfat, Wasserstoffperoxid oder organische Peroxide, z.B. tert.-Butylhydroperoxid.

Geeignet sind insbesondere sogenannte Reduktions-, Oxidations(Red-Ox)-Initiator Systeme.

Die Red-Ox-Initiator-Systeme bestehen aus mindestens einem meist organischem Reduktionsmittel und einem anorganischen oder organischen Oxidationsmittel.

Bei der Oxidationskomponente handelt es sich z.B. um die bereits vorstehend genannten Initiatoren für die Emulsionspolymerisation.

Bei den Reduktionskomponenten handelt es sich z.B. um Alkalimetallsalze der schwefligen Säure, wie z.B. Natriumsulfit, Natriumhydrogensulfit, Alkalisalze der dischwefligen Säure wie Natriumdisulfit, Bisulfitadditionsverbindungen aliphatischer Aldehyde und Ketone, wie Acetonbisulfit oder Reduktionsmittel wie Hydroxymethansulfinsäure und deren Salze oder Ascorbinsäure. Die Red-Ox-Initiator-Systeme können unter Mitverwendung löslicher Metallverbindungen, deren metallische Komponente in mehreren Wertigkeitsstufen auftreten kann, verwendet werden.

Übliche Red-Ox-Initiator-Systeme sind z.B. Ascorbinsäure/Eisen(II)sulfat/Natriumperoxodisulfat, tert.-Butylhydroperoxid/Natriumdisulfit, tert.-Butylhydroperoxid/Na-Hydroxymethansulfinsäure. Die einzelnen Komponenten, z.B. die Reduktionskomponente, können auch Mischungen sein z.B. eine Mischung aus dem Natriumsalz der Hydroxymethansulfinsäure und Natriumdisulfit.

Die genannten Verbindungen werden meist in Form wäßriger Lösungen eingesetzt, wobei die untere Konzentration durch die in der Dispersion vertretbare Wassermenge und die obere Konzentration durch die Löslichkeit der betreffenden Verbindung in Wasser bestimmt ist. Im allgemeinen beträgt die Konzentration 0,1 bis 30 Gew.-%, bevorzugt 0,5 bis 20 Gew.-%, besonders bevorzugt 1,0 bis 10 Gew.-%, bezogen auf die Lösung.

Die Menge der Initiatoren beträgt im allgemeinen 0,1 bis 10 Gew.-%, bevorzugt 0,5 bis 5 Gew.-%, bezogen auf die zu polymerisierenden Monomeren. Es können auch mehrere, verschiedene Initiatoren bei der Emulsionspolymerisation Verwendung finden.

Bei der Polymerisation können Regler eingesetzt werden, z.B. in Mengen von 0 bis 0,8 Gew.-Teile, bezogen auf 100 Gew.-Teile der zu polymerisierenden Monomeren, durch die die Molmasse verringert wird. Geeignet sind z.B. Verbindungen mit einer Thiolgruppe wie tert.-Butylmercaptan, Thioglycolsäureethylhexylester, Mercaptoethanol, Mercaptopropyltrimethoxysilan oder tert.-Dodecylmercaptan. Die Regler enthalten keine polymerisierbare, ethylenisch ungesättigte Gruppe. Die Regler bewirken einen Abbruch der Polymerisationskette und werden daher endständig an die Polymerketten gebunden.

Die Emulsionspolymerisation erfolgt in der Regel bei 30 bis 130, vorzugsweise 50 bis 90°C. Das Polymerisationsmedium kann sowohl nur aus Wasser, als auch aus Mischungen aus Wasser und damit mischbaren Flüssigkeiten wie Methanol bestehen. Vorzugsweise wird nur Wasser verwendet. Die Emulsionspolymerisation kann sowohl als Batchprozeß als auch in Form eines Zulaufverfahrens, einschließlich Stufen- oder Gradientenfahrweise, durchgeführt werden. Bevorzugt ist das Zulaufverfahren, bei dem man einen Teil des Polymerisationsansatzes vorlegt, auf die Polymerisationstemperatur erhitzt, anpolymerisiert und anschließend den Rest des Polymerisationsansatzes, üblicherweise über mehrere räumlich getrennte Zuläufe, von denen einer oder mehrere die Monomeren in reiner oder in emulgierter Form enthalten, kontinuierlich, stufenweise oder unter Überlagerung eines Konzentrationsgefälles unter Aufrechterhaltung der Polymerisation der Polymerisationszone zuführt. Bei der Polymerisation kann auch z.B. zur besseren Einstellung der Teilchengröße eine Polymersaat vorgelegt werden.

Die Art und Weise, in der der Initiator im Verlauf der radikalischen wäßrigen Emulsionspolymerisation dem Polymerisationsgefäß zugegeben wird, ist dem Durchschnittsfachmann bekannt. Es kann sowohl vollständig in das Polymerisationsgefäß vorgelegt, als auch nach Maßgabe seines Verbrauchs im Verlauf der radikalischen wäßrigen Emulsionspolymerisation kontinuierlich oder stufenweise eingesetzt werden. Im einzelnen hängt dies in an sich dem Durchschnittsfachmann bekannter Weise sowohl von der chemischen Natur des Initiatorsystems als auch von der Polymerisationstemperatur ab. Vorzugsweise wird ein Teil vorgelegt und der Rest nach Maßgabe des Verbrauchs der Polymerisationszone zugeführt.

Zur Entfernung der Restmonomeren wird üblicherweise auch nach Ende der eigentlichen Emulsionspolymerisation, d.h. nach einem Umsatz der Monomeren von mindestens 95 %, Initiator zugesetzt.

Die einzelnen Komponenten können dem Reaktor beim Zulaufverfahren von oben, in der Seite oder von unten durch den Reaktorboden zugegeben werden.

Bei der Emulsionspolymerisation werden wäßrige Lösungen oder Dispersionen des Polymeren in der Regel mit Feststoffgehalten von 15 bis 75 Gew.-%, bevorzugt von 25 bis 60 Gew.-% erhalten.

Die erhaltenen Copolymerisate werden dann teilweise oder vollständig in die Salzform überführt.

Dazu werden bereits während oder nach der Polymerisation basische anorganische Salze z.B. Hydroxide, Oxide, Phosphate, Carbonate, Hydrogencarbonate, Acetate oder Formiate der Alkali- oder Erdalkalimetalle oder des Zinks zugesetzt. Bevorzugt verwendet man die Oxide und Hydroxide.

Die Methoden der Neutralisation d.h. der Überführung von Carboxylgruppen enthaltenden Polymere in die Salze sind an sich bekannt und z.B. in US-A 5,037,924, Beispiel 3 beschrieben. Eine technisch vorteilhafte Methode wird in EP-A 03 15 015 beschrieben, auf die Bezug genommen wird und deren Angaben als hier inkorporiert gelten sollen.

Nach einer bevorzugten Vorgehensweise wird die wäßrige Polymerlösung oder -dispersion vorgelegt und das anorganische Salz in pulverisierter Form portionsweise in der gewünschten Menge und Zusammensetzung eingetragen. Vor der anschließenden Trocknung können gegebenenfalls weitere Zusätze zugegeben werden. Prinzipiell ist es jedoch auch möglich, das nichtneutralisierte Copolymerisat zu trocknen und anschließend mit dem anorganischen Salz zu versetzen.

In Abhängigkeit vom Anteil an Acrylsäure bzw. Methacrylsäure d.h. der Zahl der Carboxylgruppen im Copolymerisat und vom Neutralisationsgrad erhält man entweder eine Polymerdispersion oder eine wäßrige Polymerlösung, die zur Herstellung der Haftmittel getrocknet werden, um mit dem Träger (b) zum fertigen Haftmittel verarbeitet werden zu können.

Die Trocknung erfolgt mit an sich bekannten Methoden z.B. in Wirbelschichttrocknern oder bevorzugt durch Sprühtrocknung. Es kann sich dabei als zweckmäßig erweisen, den Feuchtigkeitsgehalt des getrockneten Produkts so einzustellen, daß eine Restfeuchte von bis zu 10 Gew.-% bestehen bleibt, wie dies in DE-A 21 33 709 beschrieben ist. Ferner hat es sich als zweckmäßig erwiesen, bestimmte Teilchengrößen des getrockneten Copolymers z.B. von weniger als 250 µ, vorzugsweise von 5 bis ungefähr 200 µ einzuhalten, d. h. gegebenenfalls kann eine Siebung oder Mahlung erforderlich sein.

Die Compoundierung des Copolymers (a) mit dem Träger (b) erfolgt in üblicher Weise durch mechanisches Einarbeiten bei Temperaturen unter 50°C. Dabei können weitere Zusätze, die die Haftkraft der Massen nicht beeinträchtigen miteingearbeitet werden, wie Geschmacksstoffe, Verdicker wie Kieselsäurepulver oder Farbstoffe.

Die Trägerkomponente (b)enthält, in der Regel als wesentliche Bestandteile ein Mineralöl wie Paraffinöl, oder Vaseline wie Petrolatum und gegebenenfalls die Viskosität regelnde Zusätze wie Polyethylenglykol oder Glycerin oder niedermolekulares Polyethylen. Das Mineralöl mit der erforderlichen Viskosität ist bevorzugt ein hochraffiniertes Weißöl mit einer Viskosität von 50 bis 350 mPas bei 38°C. Das niedermolekulare Polyethylen hat ein Molekulargewicht von z.B. 1000 bis 21 000, vorzugsweise 2000 bis 5000, gemessen mit Gelpermeationschromatographie. Pulverförmige Polyethylenpolymere mit einem mittleren Molekulargewicht von ungefähr 2000 sind besonders bevorzugt. Der Anteil des Trägers (b) in der fertigen Haftmasse beträgt in der Regel 0,1 bis 60 Gew.-%.

Prinzipiell kann auf die Verwendung von Ölen in den Zahnprothesenhaftmitteln verzichtet werden, wenn beispielsweise ein Haftpulver hergestellt werden soll.

Den Copolymeren (a) können vor oder nach der Trocknung, oder auch der fertigen Mischung aus (a) und dem Träger (b) noch weitere haftaktive Stoffe oder die sonstigen Eigenschaften der Prothesenhaftmittel verbessernde Zusätze zugefügt werden, wie an sich bekannte Alkylvinylether-Maleinsäureanhydrid-Copolymerisat-Partialsalze, Natriumcarboxymethylcellulose, Polyethylenoxid, Kharayagummi, Polyethylenglykol, Natriumalginat, Hydroxyethylcellulose, Chitosan, Guargummi, Carbopolpolymere, Polyvinylalkohol, Polyvinylpyrrolidon, Copolymerisate aus Vinylpyrrolidon und Vinylacetat, Polysaccharide oder chemisch modifizierte Polysaccharide oder weitere dem Fachmann bekannte haftaktive Stoffe.

Die neuen Zahnprothesenhaftmittel können zusätzlich, wie in EP-A 00 73 850 beschrieben gecoatet werden, um auch die Haftdauer mit verzögerter Verfügbarkeit der haftaktiven Massen zu erhöhen.

Die erfindungsgemäßen Zahnprothesenhaftmittel zeichnen sich durch verbesserte Haftkraft und sehr einfache Herstellung und physiologische Unbedenklichkeit aus.

Bestimmte Copolymerisate (a) sind schon in nicht neutralisierter Form und auch mit Alkalizusatz aus EP-A 0 403 959 und DE-A 3 208 791 als Tablettenüberzugsmittel mit kontrollierter Löslichkeit bzw. Quellbarkeit im Magen-Darm-Trakt bekannt.

Ferner ist aus USA 4,529,748 bekannt, nicht neutralisiertes Poly[methacrylsäure, methylmethacrylat 1.2] als Überzug auf Nacarboxymethylcellulose in Zahnprothesenhaftmitteln zu verwenden mit dem Ziel durch allmähliches Auflösen des Überzugs frisches Haftmittel in Form der Na-carboxymethylcellulose freizusetzen und damit die Haftdauer zu erhöhen. Die erfindungsgemäße Verwendung der zumindest teilweise neutralisierten Carboxylgruppen enthaltenden Copolymere als haftaktiven Wirkstoff wird dadurch weder beschrieben noch nahegelegt.

### Beispiele

### Beispiel 1 (Herstellung der Copolymerisate (a))

Aus 1 g Natriumlaurylsulfat 6,7 g eines handelsüblichen nichtionischen Emulgators, 100 g Wasser, 1,3 g Ethylhexylthioglycolat und 300 g Monomermischung stellte man eine Emulsion her, die im Zulaufverfahren in ein Polymerisationsgefäß, das 500 g Wasser enthielt, im Laufe der Polymerisation innerhalb von ca. 2 bis 4 Stunden bei ca. 75 bis 85°C gegeben wurde. Der Initiator, 1 g Natriumpersulfat gelöst in 100 g Wasser, wurde ebenfalls im Laufe der Polymerisation kontinuierlich zugeführt. Zur Senkung des Restmonomerengehalts wurde anschließend mit einem Redoxinitiator (tert.-Butylhydroperoxid/Ascorbinsäure) versetzt und nachpolymerisiert.

Zur Herstellung des Polymerisalzes wurde anschließend mit Wasser verdünnt und mit den erforderlichen Mengen an Oxiden oder Hydroxiden versetzt.

Die erhaltenen Polymerlösungen bzw. -Dispersionen wurden schließlich sprühgetrocknet.

Die so hergestellte Copolymeren sind in der folgenden Tabelle 1 aufgeführt.

**Tabelle 1**

| Beispiel | Monomere | Monomerverhältnis | Salz | Neutralisationsgrad |
|---|---|---|---|---|
| a | EA/MAS | 1:1 | Na | 35 % |
| b | EA/MAS | 1:1 | Na/Ca=1/6 | 35 % |
| c | EA/MAS | 1:1 | Ca | 40 % |
| d | EA/MAS | 1:1 | Na/Zn=1/3 | 40 % |
| e | EA/MAS | 1:1 | Zn | 40 % |
| f | MMA/MAS | 2:1 | Na | 95 % |
| g | MMA/MAS | 2:1 | Na/Ca=1/6 | 85 % |
| h | MMA/MAS | 2:1 | Na/Sr=1/3 | 85 % |
| i | tBA/MAS | 1:1 | K | 60 % |
| j | MMA/MAS | 1:1 | Na/Zn=1/4 | 45 % |
| EA = Ethylacrylat MAS = Methacrylsäure MMA = Methylmethacrylat tBA = tert.-Butylacrylat | | | | |

### Beispiel 2

### (Herstellung des Zahnprothesenhaftmittels)

40 Teile der sprühgetrockneten Pulver 1a bis 1j (Teilchengröße 5 bis 200µ, Wassergehalt 3,0 Gew.-%) wurden mit 20 Teilen Paraffinöl (110 - 230 mPas) und 40 Teilen Vaseline (Petrolatum der Firma Riedel de Haen) bei 25°C innig vermischt.

### Beispiel 3

### (Vergleich der Haftwirkung mit bekannten Zahnprothesenhaftmitteln)

Zur Bestimmung der Wirksamkeit von Zahnprothesenhaftklebern wurden zwischen zwei Polymethylmethacrylat-Formteilen jeweils 0,3 g der zu untersuchenden Formulierung möglichst gleichmäßig aufgetragen und die Zugspannung bestimmt, die zur Trennung der Formteile erforderlich ist.

Die Zugspannung wurde für alle Beispiele 1a bis 1j innerhalb von ca. 2 min zu 2700 bis ca. 3000 cN bestimmt. Für vorbekannte Haftcremes wurden in keinem Fall Zugspannungen von mehr als 2640 cN gemessen.

## Patentansprüche

1. Zahnprothesenhaftmittel enthaltend
a) als haftaktiven Wirkstoff teilweise oder vollständig neutralisierte Copolymerisate aus
5 bis 95 Gew.-%, bezogen auf das Copolymerisat, eines oder mehrerer Acrylester der Formel I
CH₂=CR'―COOR I (Monomer A),
in der R' Wasserstoff oder Methyl und R einen Alkylrest mit 1 bis 30 C-Atomen bedeutet und
95 bis 5 Gew.-% Methacrylsäure und/oder Acrylsäure (Monomer B), und
b) einen in Zahnprothesenhaftmitteln üblichen Träger.

2. Zahnprothesenhaftmittel gemäß Anspruch 1, dadurch gekennzeichnet, daß das Copolymerisat wasserlöslich ist.

3. Zahnprothesenhaftmittel gemäß Anspruch 1, dadurch gekennzeichnet, daß das Copolymerisat als einheitliches oder Mischsalz des Natriums, Kaliums, Calciums, Magnesiums, Strontiums oder Zinks vorliegt.

4. Zahnprothesenhaftmittel gemäß Anspruch 1, dadurch gekennzeichnet, daß 30 bis 95 % der freien Carboxylgruppen neutralisiert sind.

5. Zahnprothesenhaftmittel gemäß Anspruch 1, dadurch gekennzeichnet, daß die Monomere (A) C₁- bis C₄-Ester der Acrylsäure oder Methacrylsäure sind.

6. Zahnprothesenhaftmittel gemäß Anspruch 1, dadurch gekennzeichnet, daß die Copolymerisate 0 bis 30 Gew.-%, bezogen auf das Copolymerisat, weitere radikalisch polymerisierbare Monomere (Monomer C), ausgewählt aus der Gruppe bestehend aus Acrylamiden, Methacrylamiden, N-Vinyllaktamen, Hydroxialkylacrylsäureestern, Crotonsäure oder Maleinsäure enthalten.

7. Zahnprothesenhaftmittel gemäß Anspruch 1, dadurch gekennzeichnet, daß sie als haftaktive Masse zusätzlich zu (a) an sich bekannte haftaktive Massen ausgewählt aus der Gruppe, bestehend aus Partialsalzen von Alkylvinylether-Maleinsäure-Copolymerisaten, Carboxymethylcellulose und Salzen hiervon, Polyvinylpyrrolidon oder Copolymerisaten von Vinylpyrrolidon und Vinylacetat, Alginaten, Polyethylenglycolen, Polyethylenglycolcopolymeren oder deren Mischungen enthalten.

8. Zahnprothesenhaftmittel gemäß Anspruch 1, dadurch gekennzeichnet, daß sie als haftaktive Masse (a) ein Copolymerisat aus
20 bis 80 Gew.-%, bezogen auf das Copolymerisat, Ethylacrylat oder Methylmethacrylat und
80 bis 20 Gew.-% Methacrylsäure oder Acrylsäure enthalten.

9. Verwendung von Copolymerisaten aus
5 bis 95 Gew.-% eines Monomers der Formel
CH₂=CR'―COOR I (Monomer A),
in der R' Wasserstoff oder eine Methylgruppe und R einen Alkylrest mit 1 bis 30 C-Atomen bedeutet, und
95 bis 5 Gew.-% Methacrylsäure und Acrylsäure (Monomer B), mit einem Molekulargewicht von 30.000 bis 5 Mio. Dalton, wobei die freien Carboxylgruppen des Copolymerisats teilweise oder vollständig neutralisiert sind, als haftaktive Masse, zur Herstellung von Zahnprothesenhaftmitteln.

10. Verfahren zur Herstellung von Zahnprothesenhaftmitteln gemäß Anspruch 1, dadurch gekennzeichnet, daß man
(i) Monomere A der Formel I
CH₂=CR'―COOR I (Monomer A),
in der R' Wasserstoff oder Methylgruppe und R Alkyl mit 1 bis 30 C-Atomen bedeutet, mit Monomeren B ausgewählt aus der Gruppe bestehend aus Methacrylsäure oder Acrylsäure copolymerisiert, wobei das Gewichtsverhältnis von Monomer A zu Monomer B 95 zu 5 bis 5 zu 95 beträgt,
(ii) das erhaltene Copolymerisat vollständig oder teilneutralisiert,
(iii) das neutralisierte Copolymerisat trocknet und
(iv) mit einem Träger in an sich bekannter Weise zu dem Prothesenhaftmittel mischt und die erhaltene Mischung gegebenenfalls
(v) in die Pulverform überführt.
